# EUROPEAN PATENT APPLICATION

(11) **EP 2 060 235 A1**
(43) Date of publication of application: **20.05.2009**
(21) Application number: 08002430.0
(22) Date of filing: 11.02.2008
(51) Int. Cl.: A61B 17/34

(54) **Surgical Trocar with separate access channel**

(30) Priority: 16.11.2007 US 941258
(71) Applicant: HOLLIS INNOVATIONS L.L.C., Lebanon TN 37087 (US)
(72) Inventor: Hollis, Jeffrey D., Gallatin, TN 37066 (US); Hollis, Greg, Nashville, TN 37212 (US)
(74) Representative: Moinas, Michel

(57) **Abstract**

The present invention provides a surgical trocar (10) including an elongated trocar passageway (40) and an access channel (60) disposed in spaced relationship to each other. The access channel (60) includes a gas seal (66) to prevent gas leakage while the access channel (60) is in use, and a valve (70) to close the access channel (60) when not in use. Once, the trocar (10) is inserted into the body cavity, the access channel (60) allows access of catheters and or other small diameter devices without the need for making extra incisions. This allows continued use of the trocar (10) while utilizing the access channel (60), giving it a dual function usage. The access channel (60) allows devices to be angled away from the trocar (10) once inside the body cavity making device manipulation easier.

## Description

### BACKGROUND OF THE INVENTION

### Field of the Invention

The present invention relates to the field of surgical instruments, and more particularly to a surgical trocar having an access channel disposed in spaced relationship to the trocar passageway.

### Description of Related Art

As can be seen by reference to the following U.S. Patent Nos. 5,139,487; 5,743,881; 5,941,852; U.S. Publn. 20070016100; and EP0756505, the prior art is replete with myriad and diverse surgical trocars.

While all of the aforementioned prior art constructions are adequate for the basic purpose and function for which they have been specifically designed, they are uniformly deficient with respect to their failure to provide a simple, efficient, and practical trocar having an integrated access channel.

As a consequence of the foregoing situation, there has existed a longstanding need for a new and improved surgical trocar, and the provision of such a device is a stated objective of the present invention.

### BRIEF SUMMARY OF THE INVENTION

Briefly stated, the present invention provides a surgical trocar including an elongated trocar passageway and an access channel disposed in spaced relationship to each other. The access channel includes a gas seal to prevent gas leakage while the access channel is in use, and a valve to close the access channel when not in use. Once, the trocar is inserted into the body cavity, the access channel allows access of catheters and or other small diameter devices without the need for making extra incisions. This allows continued use of the trocar while utilizing the access channel, giving it a dual function usage. The access channel allows devices to be angled away from the trocar once inside the body cavity making device manipulation easier.

One embodiment of the trocar has the access channel integrally formed in a housing with the trocar passageway. Another embodiment of the trocar includes a sheath that receives the elongated housing of a conventional trocar where the sheath includes an access channel disposed in spaced relationship to the trocar passageway.

The objective of this invention is to provide a multipurpose access channel for a trocar, for use during laparoscopic surgery. Current laparoscopy trocars do not provide an access channel and thus require separate incisions if a small diameter device, such as a catheter, is to be used. This improves current trocars by making them more versatile and eliminates the need for excess incisions Currently, during laparoscopic cholecystectomy, a separate stab incision is required to insert a cholangiocatheter or stone retrieval device. This new access channel provides integrated access through the existing trocar, removing the need for extra incisions.

### BRIEF DESCRIPTION OF THE SEVERAL VIEWS OF THE DRAWINGS

These and other attributes of the invention will become more clear upon a thorough study of the following description of the best mode for carrying out the invention, particularly when reviewed in conjunction with the drawings, wherein:
FIG. 1 is a side elevational view of the surgical trocar of the present invention, with the proximal and distal ends cut away;
FIG. 2 is an enlarged partial sectional view of the proximal end of the trocar;
FIG. 3 is an enlarged partial sectional view of the distal end of the trocar;
FIG. 4 is a proximal end elevational view of the trocar;
FIG. 5 is a distal end elevational view of the trocar;
FIG. 6 is a side elevational view of a glove sheath for a conventional trocar, with the proximal and distal ends cut away;
FIG. 7 is an enlarged partial sectional view of the proximal end of the sheath;
FIG. 8 is an enlarged partial sectional view of the distal end of the sheath;
FIG. 9 is a proximal end elevational view of the sheath; and
FIG. 10 is a distal end elevational view of the sheath.

### DETAILED DESCRIPTION OF THE INVENTION

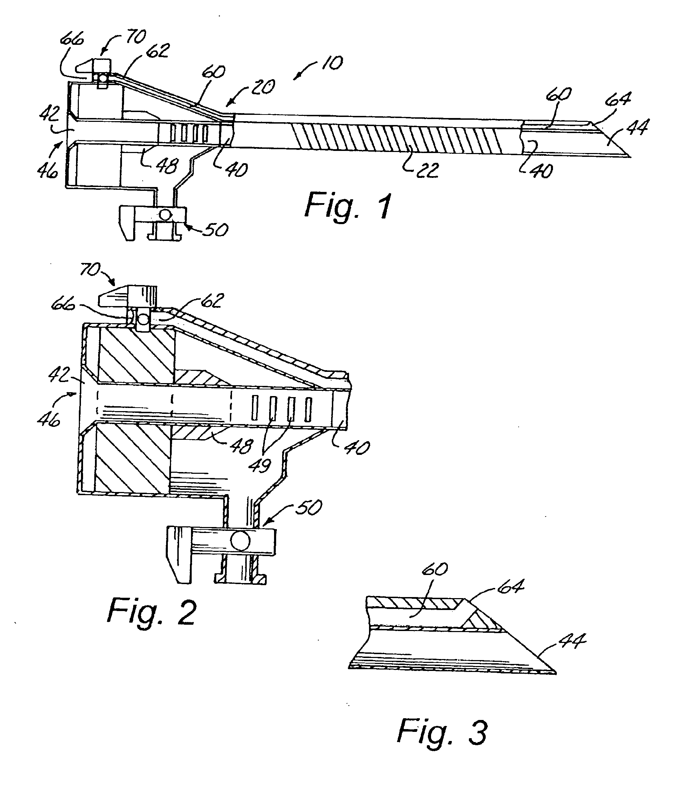

As can be seen by reference to the drawings, and in particular to FIG. 1, the surgical trocar that forms the basis of the present invention is designated generally by the reference number **10.** The trocar **10** includes an elongated housing **20** with a trocar passageway **40** and an access channel **60** formed in the housing **20** in spaced relationship to each other. The exterior of the housing **20** has grooves **22** for tissue traction.

The trocar passageway **40** has a proximal end **42** and a distal end **44.** The proximal end **42** is in open communication with the trocar collar **46** which includes a rubber boot gas seal **48** through which the instrument (not shown) is directed and internal gas inlets **49.** A stopcock valve **50** is also in communication with the trocar passageway **40.**

The access channel **60** has a proximal end **62** and a distal end **64.** A diaphragm gas seal **66** is disposed near the proximal end **62** to prevent gas leakage out through the access channel **60** when it is in use. Also, a stopcock valve **70** is provided to close the access channel **60** when it is not in use. A portion of the access channel **60** at the distal end **64** is directed away from the longitudinal axis of the trocar passageway **40** so that devices carried through the access channel **40** may be easily manipulated without interference with the instrument introduced through the trocar passageway **40.**

The invention may be used for different small diameter devices on different size trocars. The trocar function itself is not compromised. This access channel **60** can be used for diagnostic and therapeutic interventions depending on the device. Diagnostic uses, for example, would include a channel for cholangiography or liver biopsy. Therapeutic uses, for example, would include catheters for bile duct exploration such as stone baskets.

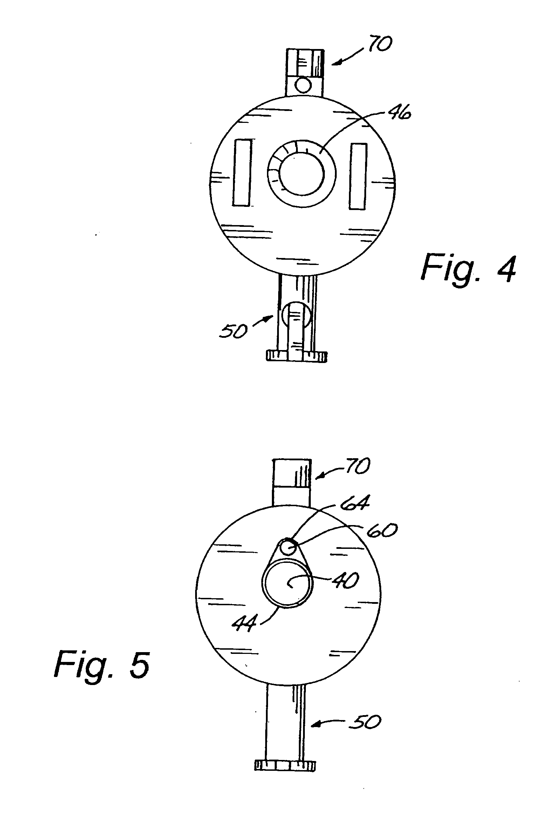

The trocar may be structured differently by changing the location of the access channel **60** either longitudinally or obliquely in reference to the trocar passageway **40.** The access channel **60** can be structured to different diameters to accommodate different interventional devices. The opening of the channel **60** can be structured to open at the distal end **44** or anywhere along the trocar passageway **40.** The trocar itself can be structured to different sizes depending on what access to a body cavity is needed. Multiple access channels can be placed on a single trocar. The channel can be incorporated into either cutting or dilating type trocars. The cross section of the housing **20** could be modified from the tear drop shape shown in FIG. 5 without changing the functionality.

The device works by not only allowing laparoscopic access to a body cavity, but also by providing a built in channel **60** for small diameter device access. The trocar passageway **40** allows laparoscopy instruments to be inserted into a body cavity for surgery. The integrated access channel **60** allows simultaneous use of the trocar **10** for other small diameter devices, such as cholangiocatheters, without compromising the use of the trocar passageway **40** for instruments. This design provides a versatile alternative to current trocars.

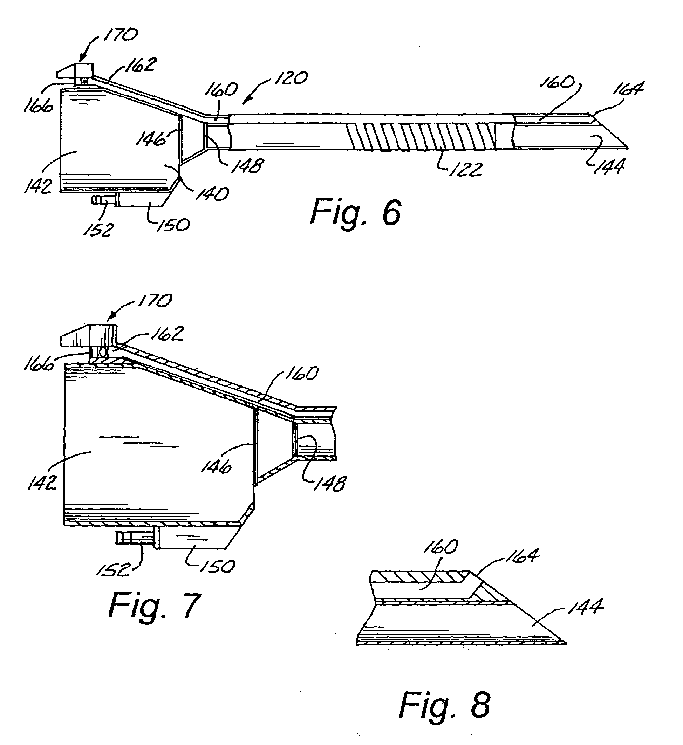

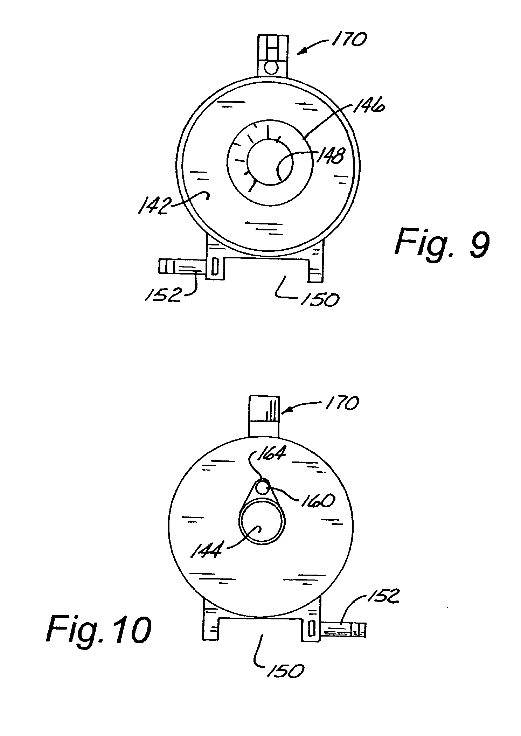

A second embodiment of the invention uses a glove sheath **120** as shown in FIGS. 6-10, in combination with a conventional trocar. The sheath **120** is disposed to matingly receive the housing of a conventional trocar. The sheath **120** includes a trocar receiving cavity **140** and an access channel **160** disposed in spaced relationship to the cavity **140.** The exterior of the sheath **120** has grooves **122** for tissue traction.

The trocar cavity **140** has a proximal end **142** and a distal end **144.** A pair of "O"-ring seals **146** and **148** prevents gas leakage and stabilizes the trocar within the cavity **140.** A groove **150** receives the air insufflation port of the trocar, and a latch **152** secures the trocar within the sheath **120** at the air insufflation point.

The access channel **160** has a proximal end **162** and a distal end **164.** A diaphragh gas seal **166** and a stopcock valve **170** are carried near the proximal end **162.** The sheath **120** thus provides a retrofit of a conventionl trocar that functions like the main embodiment of the present invention.

Although only an exemplary embodiment of the invention has been described in detail above, those skilled in the art will readily appreciate that many modifications are possible without materially departing from the novel teachings and advantages of this invention. Accordingly, all such modifications are intended to be included within the scope of this invention as defined in the following claims.

Having thereby described the subject matter of the present invention, it should be apparent that many substitutions, modifications, and variations of the invention are possible in light of the above teachings. It is therefore to be understood that the invention as taught and described herein is only to be limited to the extent of the breadth and scope of the appended claims.

## Claims

1. A surgical trocar comprising:
an elongated housing;
an elongated trocar passageway formed in the housing, the passageway having a proximal end, a distal end, and a longitudinal axis; and
an elongated access channel disposed in spaced relationship to the trocar passageway, the access channel having a proximal end and a distal end.

2. The surgical trocar of claim 1, further including a gas seal disposed within the access channel near its proximal end, whereby gas is prevented from exiting its proximal end while the access channel is in use.

3. The surgical trocar of claim 1 or 2, further including a valve disposed within the access channel near its proximal end, whereby the access channel may be closed when not in use.

4. The surgical trocar of any one of claims 1 to 3, wherein the distal end of the access channel is inwardly spaced from the distal end of the trocar passageway.

5. The surgical trocar of any one of claims 1 to 4, wherein a portion of the access channel adjacent its distal end is directed away from the longitudinal axis of the trocar passageway.

6. The surgical trocar of any one of claims 1 to 5, wherein the access channel is formed in the housing.

7. The surgical trocar of any one of claims 1 to 5, wherein the access channel is formed in a sheath disposed to receive the elongated housing of the trocar.

8. The surgical trocar of any one of claims 1 to 7, wherein the housing has a tear drop cross section.
